(19) **Europäisches Patentamt**
**European Patent Office**
**Office européen des brevets**

(11) **EP 0 975 351 B1**

(12) **EUROPEAN PATENT SPECIFICATION**

(45) Date of publication and mention
of the grant of the patent:
**14.03.2007 Bulletin 2007/11**

(51) Int Cl.:
**A61K 9/00** *(2006.01)*     **A61K 9/28** *(2006.01)*
**A61K 38/16** *(2006.01)*

(21) Application number: **98901745.4**

(22) Date of filing: **12.01.1998**

(86) International application number:
**PCT/US1998/000375**

(87) International publication number:
**WO 1998/030227 (16.07.1998 Gazette 1998/28)**

(54) **TREATMENT OF MULTIPLE SCLEROSIS THROUGH INGESTION OF COPOLYMER-1**

BEHANDLUNG DER MULTIPLEN SKLEROSE DURCH EINNAHME VON COPOLYMER-1

TRAITEMENT DE LA SCLEROSE EN PLAQUES PAR L'INGESTION DE COPOLYMERE-1

(84) Designated Contracting States:
**AT BE CH DE DK ES FI FR GB GR IE IT LI LU MC NL PT SE**
Designated Extension States:
**AL LT LV MK RO SI**

(30) Priority: **10.01.1997 IL 11998997**

(43) Date of publication of application:
**02.02.2000 Bulletin 2000/05**

(73) Proprietor: **YEDA RESEARCH AND DEVELOPMENT CO. LTD.**
**Rehovot 76100 (IL)**

(72) Inventors:
• **ARNON, Ruth**
 **76100 Rehovot (IL)**
• **SELA, Michael**
 **76100 Rehovot (IL)**
• **TEITELBAUM, Dvora**
 **76209 Rehovot (IL)**

• **GILBERT, Adrian**
 **44448 Kfar-Saba (IL)**
• **LINENBERG, Milka**
 **40600 Tel-Mond (IL)**
• **RIVEN-KREITMAN, Rivka**
 **44288 Kfar-Saba (IL)**

(74) Representative: **MacLean, Martin Robert et al**
**Mathys & Squire**
**120 Holborn**
**London EC1N 2SQ (GB)**

(56) References cited:
**WO-A-95/31990**       **US-A- 5 719 296**

• **CHEMICAL ABSTRACTS, Vol. 125, No. 23, 02 December 1996, (Columbus, Ohio, USA), page 1, Abstract No. 291993b, JOHNSON K.P. et al., "Management of Relapsing/Remitting Multiple Sclerosis with Copolymer 1 (Copaxone)"; XP002912433 & MULT. SCLER., 1996, 1(6), 325-326 (Eng).**

**EP 0 975 351 B1**

## Description

### FIELD OF THE INVENTION

This invention relates to the use of copolymer-1 formulations for the manufacture of a medicament for the treatment of multiple sclerosis by ingestion or inhalation of glatiramer acetate as defined below). The present invention also relates to a pharmaceutical composition comprising glatiramer acetate which may be used for the treatment of multiple sclerosis, wherein the pharmaceutical composition is formulated for administration by ingestion.

### BACKGROUND OF THE INVENTION

Copolymer-1, also known as glatiramer acetate and marketed under the tradename Copaxone®, comprises the acetate salts of polypeptides containing L-glutamic acid, L-alanine, L-tyrosine and L-lysine. The average molar fraction of the amino acids are 0.141, 0.427, 0.095 and 0.338; respectively, and the average molecular weight of copolymer-1 is between 4,700 and 11,000 daltons. It is a non-autoantigen which has been demonstrated to suppress experimental allergic encephalomyelitis (EAE) induced by various encephalitogens including mouse spinal cord homogenate (MSCH) which includes all myelin antigens, such as myelin basic protein (MBP) (Sela M et al., *Bull Inst Pasteur* (1990) 88 303-314), proteolipid protein (PLP) (Teitelbaum D et al., *J Neuroimmunol* (1996) 64 209-217) and myelin oligodendrocyte glycoprotein (MOG) (Ben-Nun A et al., *J Neurol* (1996) 243 (Suppl 1) S14-S22) in a variety of species. EAE is an accepted model for multiple sclerosis.

Glatiramer acetate has been demonstrated to be active when injected subcutaneously, intra-peritoneally, intravenously or intramuscularly (D. Teitelbaum et al., *Eur.J. Immunol.* (1971) 1:242-248; D. Teitelbaum et al., *Eur. J. Immunol.* (1973) 3:273-279).

In phase III clinical trials, daily subcutaneous injections of copolymer-1 were found to slow progression of disability and reduce the relapse rate in exacerbating-remitting multiple sclerosis (K.P.Johnson, *Neurology* (1995) 1: 65-70). Glatiramer acetate therapy is presently limited to its daily subcutaneous administration.

Currently, all specifically approved treatments of multiple sclerosis involve self-injection of the active substance. Frequently observed injection-site problems include irritation, hypersensitivity, inflammation, pain and even necrosis (in the case of at least one interferon β 1-B treatment) and a low level of patient compliance. Therefore, an alternative method of administration is desirable.

EP Patent 359,783 discloses the treatment of autoimmune diseases by oral administration of autoantigens. It discloses the oral administration of MBP for treatment of multiple sclerosis. Oral administration of an autoantigen has been termed "oral tolerance."

PCT International Application Publication Nos. WO 91/12816, WO 91/08760, and WO 92/06704 all disclose the treatment of other autoimmune diseases using the "oral tolerance" method with a variety of autoantigens. However, none of these references disclose the treatment of multiple sclerosis by the oral administration of non-autoantigen glatiramer acetate. The contents of all these patents and all literature references referred to above are hereby incorporated by reference in their entirety.

It is, therefore, an object of the present invention to provide formulations for treating multiple sclerosis by oral administration of glatiramer acetate through ingestion.

The present invention provides use of an amount from 0.1 mg to 1000 mg of glatiramer acetate in a solid form in the manufacture of an enterically-coated composition for oral administration to treat multiple sclerosis.

The present invention also provides a pharmaceutical composition comprising glatiramer acetate in solid form in an amount from 0.1 mg to 1000 mg in an enterically-coated composition.

### BRIEF DESCRIPTION OF THE FIGURES

Figures 1 and 2 show the effect of glatiramer acetate on the immune response to guinea pig myelin basic protein (GPBP) in rats (Figure 1) and mice (Figure 2) as assessed by spleen cell proliferation.

Figures 3 and 4 show the effect of glatiramer acetate on cytokine release.

Figure 5 shows the suppression of EAE in mice by orally-administered glatiramer acetate. (SJL/JxBALB/c) F, mice were fed with PBS (■), 0.1 mg glatiramer acetate (□), 0.25 mg glatiramer acetate (▲), or 0.5mg glatiramer acetate (△). Each dose was fed 7 times on days -7; -5; -3; 0; 2; 4 and 6. EAE was induced on day 0 by the injection of MSCH.

Figure 6 shows the proliferation and cytokine secretion by T-cell line derived from spleens of glatiramer acetate fed rats. Cells were cultured with medium glatiramer acetate (50 $\mu$/ml) or concavalin A (Con A) (5 $\mu$g/ml). The proliferation and cytokine secretion responses to these antigens were measured.

Figure 7 shows the inhibition of EAE by T-cell line derived from spleens of glatiramer acetate fed rats. Cells ($20 \times 10^6$/rat) were injected intraperitoneally 3 days after stimulation with glatiramer acetate, followed by EAE induction.

Figure 8 shows the inhibition of EAE by T-cell line derived from spleens of glatiramer acetate fed mice. Cells ($15 \times 10^6$/mouse) were injected intravenously 3 days after stimulation with glatiramer acetate, followed by EAE induction.

FIG. 9 shows the Clinical Scores vs. Days of glatiramer acetate Post EAE Disease Induction in three Rhesus Monkeys.

FIG. 10 shows the Clinical Scores vs. Days of glatiramer acetate Post EAE Disease Induction in six Rhesus Monkeys, in a trial comparing enteric-coated vs. uncoated pharmaceutical dosage form. Values at zero (0) have been separated on the y-axis to better show the results.

## DETAILED DESCRIPTION OF THE INVENTION

Accordingly, the present invention relates to the use of glatiramer acetate for the preparation of a medicament for the treatment of multiple sclerosis. The medicament is administrated through ingestion.

The present invention is further directed to a pharmaceutical composition, which is formulated for administration through ingestion, and comprises a pharmaceutically acceptable carrier and a therapeutically effective amount of glatiramer acetate. The pharmaceutical composition is used to treat multiple sclerosis.

As stated above, glatiramer acetate comprises the acetate salts of polypeptides containing L-glutamic acid, L-alanine, L-tyrosine and L-lysine. The average molar fraction of the amino acids are 0.141, 0.427, 0.095 and 0.338, respectively, and the average molecular weight of glatiramer acetate is between 4,700 and 11,000 daltons.

The present invention is based on the observation that, for example, the oral administration of glatiramer acetate is effective in suppressing EAE, and, therefore, has a therapeutic value for the treatment of multiple sclerosis.

As contemplated, in use the glatiramer acetate is brought into contact with those lymphoid tissues in the mucosal linings which are believed to be a primary source of immune system sensitization. These mucosal linings may be found (though not necessarily exclusively) in the sinuses, trachea, bronchial passages (where they are known as the BALT or bronchi-associated lymphoid tissues) and gastrointestinal linings (known as GALT or gut-associated lymphoid tissues). Thus, glatiramer acetate is to be introduced into the body by way of ingestion. For example, glatiramer acetate may be administered by way of the mouth through feeding.

In one exemplary embodiment of the present invention, glatiramer acetate is to be introduced orally in an amount of from 0.1 to 1000 mg per day, which may be administered as a single dose or in multiple dosages. As understood by one skilled in the art, the therapeutically effective dosage is generally a function of a patient's age, sex, and physical condition, as well as a function of other concurrent treatments being administered. The determination of the optimum, therapeutically effective dosage is well within the scope of one skilled in the art.

When glatiramer acetate is to be introduced orally, it may be mixed with other food forms and consumed in solid, semi-solid, suspension, or emulsion form; and it may be mixed with pharmaceutically acceptable carriers, including water, suspending agents, emulsifying agents, flavor enhancers, and the like. In one embodiment, the oral composition is enterically-coated. Use of enteric coatings are well known in the art. For example, K. Lehman, Acrylic Coatings in Controlled Release Tablet Manufacturer, *Manufacturing Chemist and Aerosol News,* p. 39 (June 1973), and K. Lehman, Programmed Drug Release From Oral Program Forms; *Pharma. Int.,* vol. ISS 3 1971, p. 34-41, teach enteric coatings such as Eudragit S and Eudragit L. *Handbook of Pharmaceutical Excipients,* 2nd ed., also teaches Eudragit S and Eudragit L applications. One Eudragit which may be used in the present invention is L30D55.

Glatiramer acetate may be prepared by methods known in the art, for example, as disclosed in U.S. Patent No. 3,849,550, wherein the N-carboxyanhydrides of tyrosine, alanine, $\gamma$-benzyl glutamate and e-N-trifluoroacetyllysine are polymerized at ambient temperature in anhydrous dioxane with diethylamine as an initiator. The deblocking of the $\gamma$-carboxyl group of the glutamic acid is effected by hydrogen bromide in glacial acetic acid and is followed by the removal of the trifluoroacetyl groups from the lysine residues by 1M piperidine.

As described in PCT/WO95/31990, glatiramer acetate having a desired average molecular weight of about $7 \pm 2$ kilodaltons may preferably be prepared by a method comprising reacting protected glatiramer acetate with hydrobromic acid to form trifluoroacetyl glatiramer acetate, treating the trifluoroacetyl glatiramer acetate with an aqueous piperidine solution to form glatiramer acetate, and purifying the glatiramer acetate so as to result in glatiramer acetate having the desired average molecular weight.

The present invention will further be described in the examples below. However, the present invention should not be construed as being limited thereby. Unless otherwise indicated, all parts, percentages, and the like, are by weight.

## EXAMPLE 1

Antigens - Glatiramer acetate, which was prepared according to the method described in PCT/WO95/31990, was obtained from Teva Pharmaceutical Industries Ltd., Israel. GPBP was prepared from guinea pig spinal cord by acid extraction and ammonium sulfate precipitation as described in Hirshfeld H et al., *Febs Lett* (1970) 7 317-320.

Animals - (PL/xSJL/J) F1 female mice (8-10 weeks old) were obtained from Jackson Laboratories (Bar Harbor, ME). Female Lewis rats (8-12 weeks old) were obtained from Harlan-Olac (Bicester, G.B.).

Induction and Assessment of EAE - Mice were injected with 200 $\mu$g GPBP emulsified in an equal volume of complete Freund's adjuvant (CFA) containing 4 mg/ml mycobacterium tuberculosis (H37Ra) (Difco Lab, Detroit, Mich.). The emulsion at a total volume of 0.1 ml was injected into all four footpads. Immediately after and 24 hours later, pertussis toxin (250ng/mouse) (Sigma) was injected intravenously.

Rats were immunized with 25 $\mu$g of GPBP emulsified 1:1 in CFA containing 4 mg/ml. H37Ra. The emulsion at a total volume of 0.1 ml. was injected into the two hindfoot pads.

Animals were examined daily from day 10 post induction for signs of disease. EAE was scored as follows: 0-no disease, 1-limp tail, 2-hind limb paralysis, 3-paralysis of all four limbs, 4-moribund condition, 5-death.

Induction of Oral Tolerance - Mice were fed with 250 $\mu$g GPBP or glatiramer acetate dissolved in phosphate buffered saline (PBS) on days -7, -5, -3, 0, 2, 4 and 6 by gastric intubation with an 18-gauge stainless steel feeding needle (Thomas). EAE was induced on day 0.

Rats were fed with 1 mg GPBP or glatiramer acetate dissolved in PBS by gastric intubation using a sterile feeding tube (Uno Plast, Denmark). Rats were fed 5 times (total dose of 5 mg) before disease induction at intervals of 2-3 days. EAE was induced two days after the last feeding. Control mice and rats were mock fed with PBS.

Proliferation Assay - The proliferation response of spleen cells was tested 10-11 days after EAE induction as described above. Cells from 3 animals in each group were pooled and cultured in triplicate ($5\times10^5$ mouse cells and $2\times10^5$ rat cells) in microtiter plates with various antigen concentrations (GPBP) in a final volume of 0.2ml. Microtiter plates contained RPMI 1640 (available from Sigma Biochemicals, St. Louis, Missouri) culture medium supplemented with 1% autologous serum. After 72 hr. of incubation, cells were pulsed with 1 $\mu$Ci {$^3$H}-thymidine for 18 hr and then harvested onto filter papers and radioactivity was counted.

Cytokine Secretion Assay - Spleens were removed 10-11 days after EAE induction and cells of 3 mice from each group were pooled. Cells ($5\times10^6$/ml) were cultured in duplicates in 24 well plate in RPMI 1640 supplemented with 10% FCS (Fetal Calf Serum) in the presence or absence of antigen (GPBP 100 Fg/ml). Supematants were harvested after 24-40 hr of culture. Quantitative ELISA for IL-2, IFN-$\gamma$, IL-4, IL-6 and IL-10 were performed using paired mAbs specific for the corresponding cytokines (Pharmingen, La Jolla, CA) according to the manufacturer's instructions.

## Results:

The efficacy of orally administered glatiramer acetate in preventing the clinical manifestations of EAE in Lewis rats was compared to that of GPBP, when assayed under conditions previously reported to induce oral suppression by GPBP (PJ Higgins & HL Weiner, *J Immunol* (1988) 140 440-445). The results summarized in Table 1 below demonstrate that glatiramer acetate was more effective than GPBP and significantly decreased both the incidence (54% inhibition) and severity (57% inhibition) of EAE, as compared to PBS fed rats which served as control.

Table 1: Suppression of EAE in Rats by Oral Administration of glatiramer acetate.

| Fed Antigen | Incidence | Mean Maximal Score$\pm$SD | Mean Onset (days) |
|---|---|---|---|
| PBS (Control) | 27/28(96%) | 1.8$\pm$0.5 | 11.9 |
| GPBP | 10/17(59%) (p=0.0026) | 0.9$\pm$0.5 | 11.4 |
| glatiramer acetate | 13/28 (46%) (p=0.00005) | 0.78$\pm$0.45 | 12.6 |

Each figure represents the cumulative results of 3-5 independent experiments. p values represent the statistical significance of difference from the control group (Fisher exact test). Mean maximal score was calculated for the entire group.

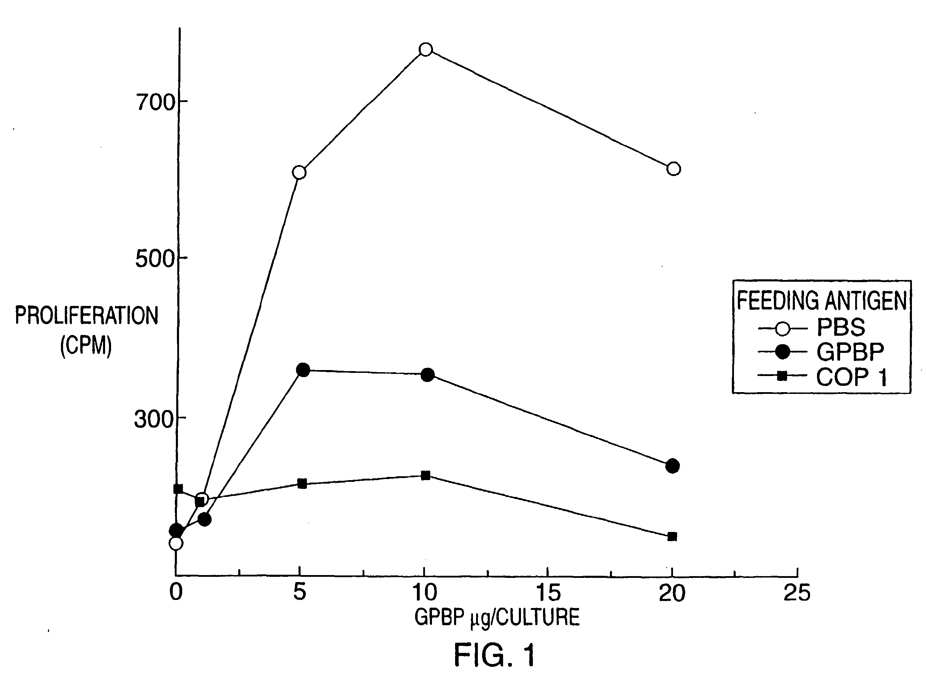

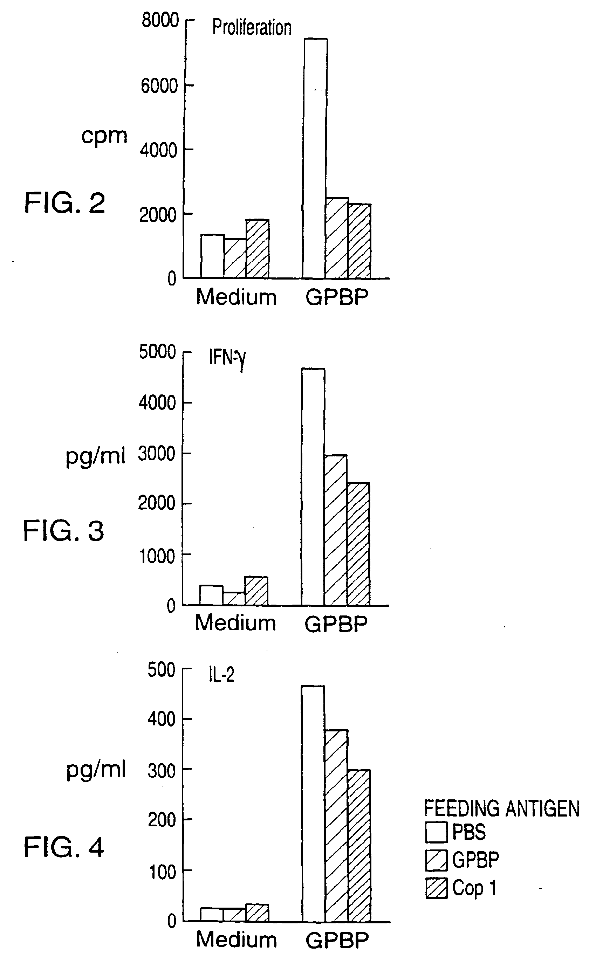

Effect of Antigen Feeding on the Immune Response to GPBP- The effect of oral administration of glatiramer acetate and GPBP on the immune response to the disease inducing antigen-GPBP was tested in mice and rats. The results are summarized in Figure 1 which shows the reduction in Cell proliferation by each of the orally administered compounds (glatiramer acetate or GPBP) in a rat spleen cell suspension stimulated with GPBP (Figure 2 shows similar results from mice). As can be seen, oral administration of glatiramer acetate resulted in almost complete inhibition of the proliferative responses to GPBP in these two species. In both species, glatiramer acetate was more effective than GPBP in inhibiting the response to GPBP.

Cytokine levels were measured in the supernatants of spleen cell cultures derived from mice (Figures 3 and 4). Control mice fed with PBS secreted IL-2; IFN-γ and IL-6 (not shown) in response to GPBP. In mice fed with glatiramer acetate or GPBP the amounts of the Th1 proinflammatory cytokines IL-2 and IFN-γ produced in response to GPBP stimulation were lower than in control groups, with glatiramer acetate being a more effective suppressant. IL-4 and IL-10 were not detected with any group treatment.

The results demonstrate that glatiramer acetate is effective in suppressing EAE when given orally. The clinically protective effect of orally administered glatiramer acetate is associated with down regulation of T cell immune responses to GPBP such as proliferation and proinflammatory cytokines (IL-2 and IFN-γ) release.

EXAMPLE 2

Additional studies on the suppression of EAE by oral administration of glatiramer acetate were performed in rats and mice. These studies established the optimal dose for treatment in each species. In order to understand the mechanism underlying oral suppression of EAE by glatiramer acetate, a glatiramer acetate specific T-cell line was isolated from spleens of glatiramer acetate fed animals. The *in vitro* reactivity of the lines and their *in vivo* effect on disease induction were studied.

Materials and Methods:

Isolation of glatiramer acetate specific T cell lines - Lewis rats were fed 5 times with 1 mg glatiramer acetate and (SJUJ x BALB/c)F, mice 7 times with 250 μg glatiramer acetate, at intervals of 2-3 days. Four to twelve days after the last feeding animals were sacrificed and their spleens removed.

Spleen cells of 3 animals were pooled and incubated ($50 \times 10^6$/plate) with glatiramer acetate (500 μg) in medium containing 1 % autologous serum for 4 days. Every 14-21 days cells ($4\text{-}6 \times 10^6$/plate) were restimulated by 3 days exposure to copolymer-1 (500 μg) presented on syngeneic irradiated (3000 rad) rat thymocytes ($100 \times 10^6$/plate) or mouse splenocytes ($50 \times 10^6$/plate). Stimulation was followed by propagation in 10% supernatant of Con A activated normal mouse spleen cells as T cell growth factor (TCGF).

Proliferation Assay - T cell lines ($1 \times 10^4$ cells) were cultured with irradiated (3000R) thymocytes (rat-$1 \times 10^6$) or splenocytes (mouse- $5 \times 10^5$) and with the indicated antigens (10 μg glatiramer acetate; 1 μg Con A) in a final volume of 0.2 ml in microtiter plates. At the end of 48 hours incubation cultures were pulsed with $^3$H-thymidine and harvested 6-12 hours later.

Cytokine Assay - T cells of rat line ($0.5 \times 10^6$/ml) were incubated with irradiated thymocytes ($10 \times 10^6$) with or without the indicated antigen (50μg glatiramer acetate; 5μg Con A). Cells were cultured for 24 hours in RPMI 1640 supplemented with 10% FCS for IL-2, TNFα, IL-4 and IL-10-measurements and in serum free medium - DCCM-1 (Biological Industries, Kibbutz Beit Haemek, Israel) for 72 hours for TGFβ measurement.

Cytokine levels in supernatants were measured in a quantitative ELISA using pairs of monoclonal antibodies specific for the corresponding cytokines.

Induction of EAE - (SJL/xBALB/c)$F_1$ mice were injected in all four footpads with 2mg mouse spinal cord homogenated (MSCH) emulsified in 1:1 ratio in CFA containing 1 mg/ml H37Ra (Difco Lab, Detroit, Mich). Pertussis toxin (250 ng/mouse, Sigma) was twice injected intravenously, once immediately after and again 48 hours later.

RESULTS:

1. Dose response study in rats and mice

Rats were fed 5 times with 0.5, 1 or 2 mg glatiramer acetate according to the established protocol (see Materials and Methods, above) and then challenged for EAE induction. The results are summarized in Table 2, and indicate that the most effective dose was 1 mg glatiramer acetate -0.5 mg or 2 mg glatiramer acetate were less efficient in suppressing EAE.

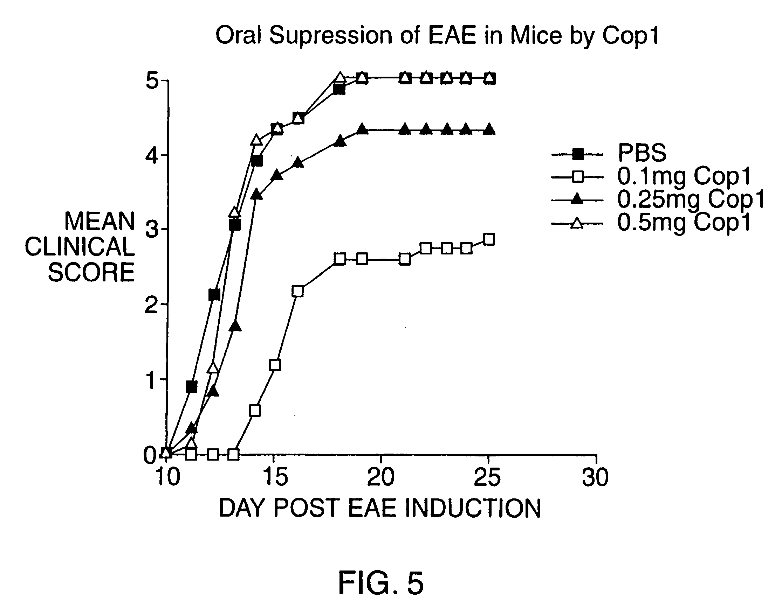

(SJL/JxBALB/c)$F_1$ mice were fed 7 times with 0.1, 0.25 or 0.5 mg glatiramer acetate on days -7; -5; -3; 0; 2; 4 and 6 by gastric intubation. EAE was induced on day 0 by the injection of MSCH. The results summarized in Figure 5 demonstrate that oral administration of glatiramer acetate could suppress the disease in mice and the most effective dose was 0.1 mg glatiramer acetate 0.25 mg of glatiramer acetate was less effective and a dose of 0.5 mg was completely inactive. Thus, the results in both rats and mice demonstrate that oral glatiramer acetate has an optimum dose response curve, and exceeding the effective oral dose resulted in inefficient suppression of EAE.

2. Studies with glatiramer acetate specific Ts-lines established from glatiramer acetate fed animals

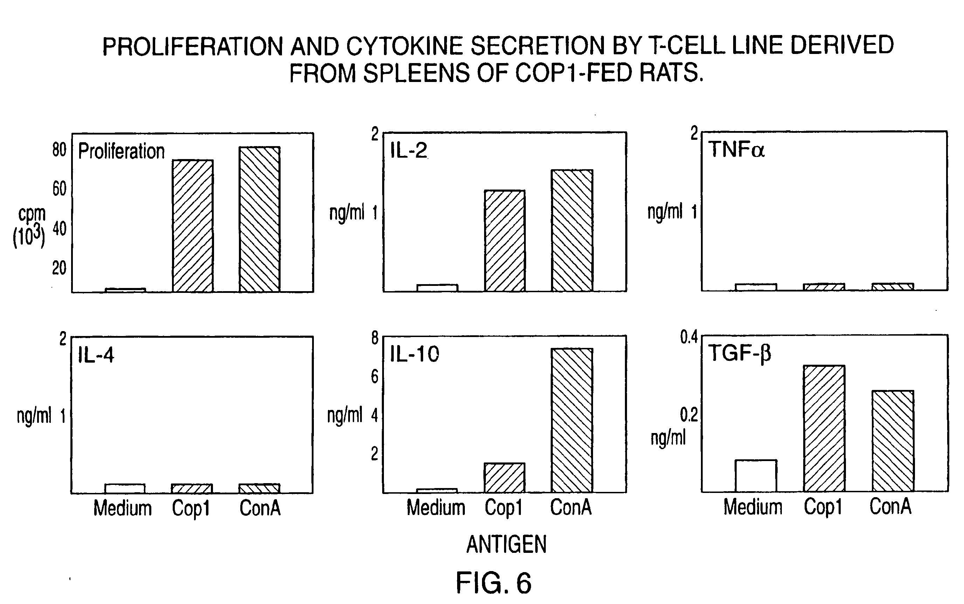

Glatiramer acetate specific T suppressor cell lines were isolated from spleens of rats and mice rendered unresponsive to EAE by feeding with glatiramer acetate. The proliferation and cytokine secretion response of such line isolated from rats is demonstrated in Figure 6. This line proliferated in response to glatiramer acetate and secreted IL-2, some IL-10 and TGFβ but not TNFα or IL-4. This cytokine profile is compatible with Th3 type cells which were shown to be induced by oral MBP (Chen et al. Science 265, 1237, 1994).

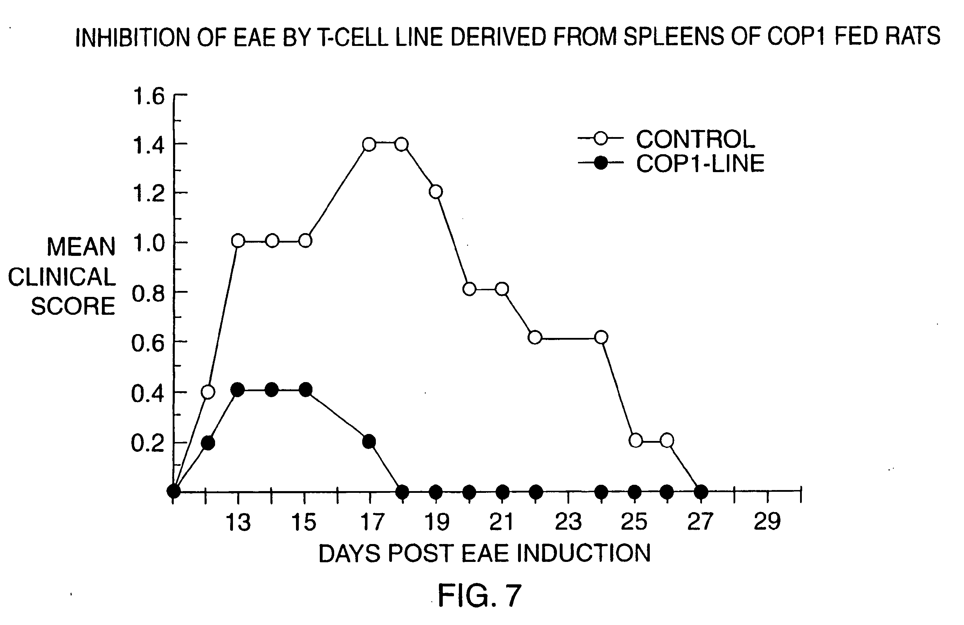

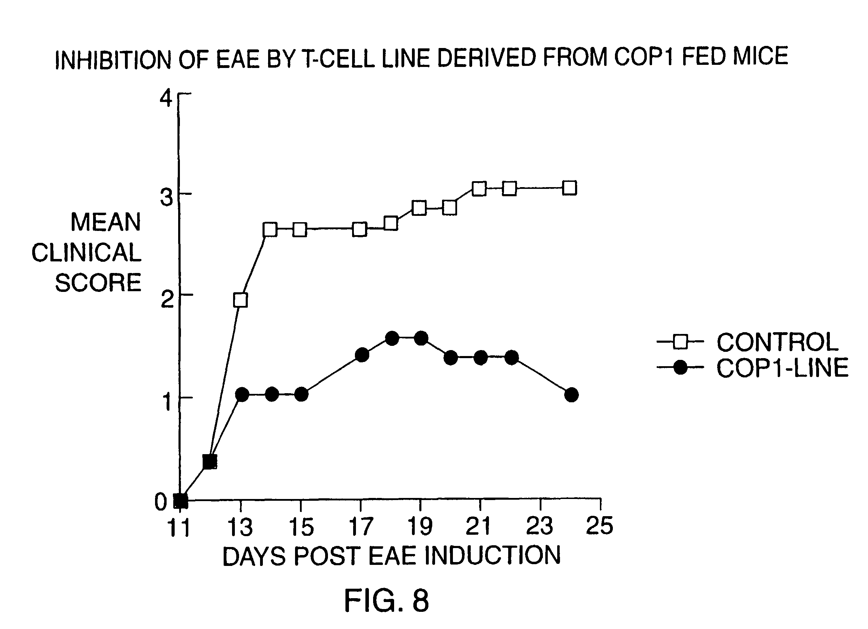

The ability of the glatiramer acetate specific lines to prevent EAE *in vivo* was studied. Cell lines were injected 3 days after *in vivo* stimulation with glatiramer acetate (20 x $10^6$ cells/rats injected i.p. and 15x$10^6$ cells/mouse injected i.v.). The animals were challenged for EAE induction immediately following cell transfer. The results illustrated in Figures 7 and 8 demonstrate that the disease was considerably inhibited in the recipient animals. Thus, both the rat and murine glatiramer acetate T-cell lines adoptively transferred the unresponsiveness to EAE induced by oral administration of glatiramer acetate. These cells actively downregulate the pathological immune response *in vivo.*

Table 2: Dose Response Study of Oral Copolymer-1 in Rats

| Fed Antigen | Incidence | Mean Score ±SD | Mean Onset | Suppression* (%) |
|---|---|---|---|---|
| PBS (control) | 10/11 | 1.32±0.64 | 13.1 | |
| glatiramer acetate (0.5 mg) | 9/11 | 0.95±0.57 | 13.5 | 28 |
| glatiramer acetate (1 mg) | 7/11 | 0.64±0.50 | 15.1 | 51 |
| glatiramer acetate (2 mg) | 8/11 | 0.91±0.70 | 13.1 | 31 |
| * calculated by mean score. | | | | |

Each incidence figure represents the cumulative results of 2 individual experiments. Mean maximum clinical score was calculated for the whole group.

Example 3

The effect of the oral administration of glatiramer acetate on the induction of EAE in Rhesus monkeys was studied.

Materials

Glatiramer acetate was provided by Teva Pharmaceutical Industries Ltd. in an enteric-coated hard gelatin capsule comprising two dosage levels: 1 mg of glatiramer acetate and 20 mg of glatiramer acetate. Each dosage level was formulated using mannitol, and coated with Eudragit L30D55. Placebo or control capsules comprised capsules containing 5 mg of sugar.

Bovine MBP was purchased from Life Technologies, Grand Island, New York. This material represents a highly purified preparation that gives a single band at 18.5 Kd following SDS-PAGE and Silver staining.

Feeding Protocol

3 Rhesus monkeys were treated as follows: One monkey served as control and was fed with placebo capsules (containing 5mg glucose only). The second and third monkeys were fed with glatiramer acetate containing capsules at a dose of 1mg/feeding and 20mg/feeding, respectively. Animals were fed every other day for a total of 10 feedings: 5 times prior to disease induction (immunization on day 0) and then 5 times after immunization.

Disease Induction

Disease was induced on day 0 by an intradermal injection of 8mg bovine - MBP and 3 mg H37Ra M. *tuberculosis* in FCA into the hind footpads, total injected volume between 0.1 and 0.15 ml per footpad. The animals were followed for clinical manifestations of EAE, a variety of serum and CSF immunological markers and spinal cord and cranial MRI's.

Clinical Scoring

Symptom scores were given as follows: 0, normal neurological exam; 1, weight loss, anorexia, yawning, slow responses to stimuli, irritability or lethargy; 2, mild neurological signs, indifference, drooling, clumsiness using limbs, tremors, altered cry and disordered gaze; 3, moderate neurological signs, blindness (pupils do not react to light), akinesia, leg weakness, or paralysis; 4, severe neurological signs, semicoma, coma, quadriplegia; 5, death.

Antigen-induced lymphocytes proliferation

Heparinized blood samples were diluted 1:1 with Hanks balanced salt solution (BSS) containing 5% heat inactivated fetal calf serum (FCS), and layered in a hypaque-ficol gradient. Centrifugation (2000 rpm for 20 minutes at room temperature) allowed recovery of diluted plasma and the separation of lymphocytes at the interface. The recovered lymphocytes were washed three times in Hanks BSS-5% FCS and resuspended in RPMI 1640 complete medium containing RPMI 1640 medium, 10% FCS, and 1% of the following reagents: non-essential amino acids, sodium pyruvate, L-glutamine, 2-mercaptoethanol, and penicillin/streptomycin. The recovered lymphocytes were counted and resuspended at a final concentration of $2 \times 10^6$/ml. Cultures containing 100 microliters of the cell suspension and 100 microliters of MBP (20 micrograms/well), 100 microliters of glatiramer acetate (10 micrograms/well) or 100 microliters of Con A (1 microgram/well) were set up in round bottom 96 well microtiter plates. The cultures were maintained at 37°C in 5% $CO_2$ for 6 days. On day 5 of culture, each well was pulsed with $1\mu$Ci of $^3$H-thymidine for 16 to 18 hours. On day 6, the cultures were harvested by an automatic cell harvester, and counted by liquid scintillation methods. Stimulation indices were determined as follows:

$$\text{Stimulation index} = \frac{\text{Experimental cpm-background cpm}}{\text{background cpm}}$$

Clinical Results

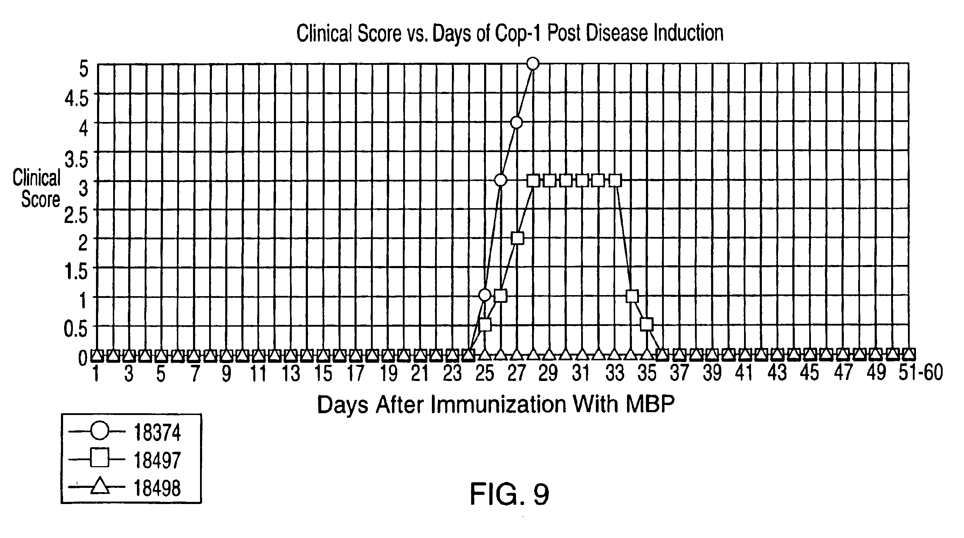

No animal exhibited clinical symptoms for 24 days. The placebo-treated animal 18374 developed disease on day 25, and due to severe manifestations of EAE (score = 4$^+$ on a scale of 5) had to be sacrificed on day 28. The high-dose glatiramer acetate treated animal 18498 treated with 20mg of copolymer-1 did not show any significant clinical symptoms over the 60 day observation period. The low-dose glatiramer acetate treated animal 18497 began to show minimal symptoms on day 25, however, in contrast with the placebo-treated animal 18374, '497 showed a much slower increase in clinical symptoms and leveled off at 2 to 3+ from day 28 to day 33. At this time-point animal '497 was fed with 20mg capsules 5 times on alternate days for a total of 10 days. As can be seen in Fig. 9, within 3 days the animal's clinical signs dropped to zero and remained there until the two glatiramer acetate fed animals '497 and '498 were sacrificed on day 60 for histology.

Flow Cytometry

The Epics C flow cytometer was used to analyze both peripheral blood lymphocytes (PBL) and cerebral spinal fluid (CSF) collected from each monkey. The red blood cells were lysed and the remaining white blood cells (WBCs) were washed and stained using standard methods with appropriate reagents. Tables 3 and 4 below show the data from staining PBL and CSF WBCs from the three animals in this Example.

The number of cells staining CD4+ was slightly greater in glatiramer acetate fed animals 18497 and 18498 than in the control animal 18374. The number of CD4+ cells that are also CD45RA- appears to increase in both control animal 18374 and low-dose glatiramer acetate fed animal 18497 about the time both animals exhibited clinical symptoms (days +27 to +34), but remained fairly constant in the high-dose glatiramer acetate fed animal 18498. The number of CD8+CD45RA+ staining cells decreased steadily in control animal 18374 and low-dose glatiramer acetate fed animal 18497, but increased slightly in high-dose glatiramer acetate fed animal 18498 (indicating the production of new CD4+ cells).

The number of cells found in the CSF of the control animal 18374 increased steadily from day 20+ until day 28+ when the animal died. Analysis showed that most of the CD4+ cells were CD45RA-. The number of cells collected from the CSF of animal 18497 were too few to count or stain. The number of cells collected from animal 18498 were too few to count or stain except on day +27. The CD4+ cells collected then were predominantly CD45RA-.

Table 3: Analysis of PBL obtained from Rhesus Monkeys immunized with MBP.

| Monkey | (Day of study) | CD4+ | CD8+ | Ratio | CD4+ CD45RA+ | CD8+ CD45RA+ |
|---|---|---|---|---|---|---|
| 18374 (control) | (-1) | 24 | 47 | 0.51 | 15 | 45 |
| | (+6) | 24 | 42 | 0.57 | 15 | 40 |
| | (+13) | 23 | 40 | 0.58 | 12 | 38 |
| | +20 | 22 | 45 | 0.49 | 10 | 45 |
| | +27 | 24 | 27 | 0.89 | 10 | 23 |
| | +28 | 24 | 24 | 1.0 | 10 | 21 |
| 1849 (1mg glatiramer acetate / dose) | -1 | 35 | 36 | 0.97 | 21 | 33 |
| | +6 | 39 | 31 | 1.26 | 24 | 29 |
| | +13 | 37 | 34 | 1.09 | 16 | 29 |
| | +20 | 41 | 32 | 1.28 | 18 | 25 |
| | +27 | 35 | 27 | 1.3 | 12 | 18 |
| | +34 | ND | ND | ND | ND | ND |
| 18498 (20 mg glatiramer acetate / dose) | -1 | 37 | 40 | 0.93 | 23 | 32 |
| | +6 | 37 | 42 | 0.88 | 23 | 32 |
| | +13 | 41 | 46 | 0.89 | 24 | 37 |
| | +20 | 27 | 57 | 0.47 | 12 | 41 |
| | +27 | 37 | 52 | 0.71 | 22 | 41 |
| | +34 | ND | ND | ND | ND | ND |
| ND=not determined | | | | | | |

Table 4: Analysis of CSF obtained from Rhesus Monkeys immunized with MBP.

| Animal | (Day of Ratio study) | Cells μl | CD4+ | CD8+ | CD4+:CD8+ Ratio | CD4+ CD45RA+ | CD8+ CD45RA+ |
|---|---|---|---|---|---|---|---|
| 18374 | 0 | ND | too few cells | | | | |
| | +14 | 28 | " | | | | |
| | +20 | 100 | 61 | 18 | 2.18 | 2 | 4 |
| | +28 | 294 | 35 | 35 | 0.94 | 4 | 8 |
| 18497 | 0 too | few cells | | | | | |
| | +14 | " | | | | | |
| | +20 | " | | | | | |
| | +27 | " | | | | | |
| | +34 | " | | | | | |

(continued)

| Animal | (Day of Ratio study) | Cells μl | CD4+ | CD8+ | CD4+:CD8+ Ratio | CD4+ CD45RA+ | CD8+ CD45RA+ |
|---|---|---|---|---|---|---|---|
| 18498 | 0 | too few cells | | | | | |
| | +14 | " | | | | | |
| | +20 | " | | | | | |
| | +27 | 29.7 | 47 | ND | | 8 | ND |
| | +34 | too few cells | | | | | |
| | +41 | | | | | | |
| ND= not determined | | | | | | | |

Analysis For Antigen-specific T cell suppressor factor inducer (Tcsfi)

The analysis, for MBP-specific Tsfi in the plasma of the three monkeys in this Example are seen in Tables 5 and 6 below. None of the animals produced MBP-specific Tsfi until day +13 following EAE induction with MBP. Control animal 18374 did not produce any Tsfi until day +20, and the level produced thereafter was just above background. Glatiramer acetate fed animals 18497 and 18498 consistently produced significant levels of Tsfi from day +13 until day +41 shortly before termination.

Table 6 shows that plasma samples which did not contain Tsfi did not react with anti-TGF-beta antibody. Plasma which exhibited MBP-binding with 3C9 antibody (anti-Tsfi) also reacted with the anti-TGF-beta antibody. Recombinant human TGF-beta reacted with the anti-TGF-beta, but not 3C9 antibody.

Table 5: Assay for MBP-specific T suppressor Inducer factor[a]

| Animal # | -13 | -1 | +6 | +13 | +20 | +27 | +34 | +41 |
|---|---|---|---|---|---|---|---|---|
| 18374 | none | none | ND | ND | .17 | .21 | died | |
| 18497 | none | none | none | .60 | .61 | .56 | .66 | .76 |
| 18498 | none | none | none | .32 | .32 | .42 | .40 | .40 |
| a. Data represents OD at 405nm, of plasma samples at a 1:20 dilution ND = Not Determined | | | | | | | | |

Table 6: The association between MBP-specific Tsfi and TGF-beta in plasma of Rhesus Monkeys treated with glatiramer acetate.

| Animal # | Day of study | anti-Tsfi (3C9 Ab)[a] | anti-TGF-beta[c] |
|---|---|---|---|
| 18374 | -1 | none | none |
| | +27 | 0.21 | none |
| 18497 | +6 | none | none |
| | +13 | 0.60 | 0.71 |
| | +27 | 0.56 | 0.65 |
| | +41 | 0.76 | 0.85 |
| 18498 | +6 | none | none |
| | +13 | 0.32 | 0.35 |
| | +27 | 0.42 | 0.47 |
| | +41 | 0.40 | 0.45 |

(continued)

| Animal # | Day of study | anti-Tsfi (3C9 Ab)[a] | anti-TGF-beta[c] |
| --- | --- | --- | --- |
|  | r human TGF-beta[c] | - | 0.72 |

a. Represents OD405 nm of material bound for MBP (2.5$\mu$g of protein) and reacts with the anti-human Tsfi (3C9) antibody.
b. Represents OD405nm of material bound to MBP and reacts with anti-human TGF-beta antibody.
c. Represents well coated with 100ng of recombinant human TGF-beta.

Example 4

Six monkeys were treated and subsequent analyses performed substantially according to the protocol described for Example 3 hereinabove.

Control placebo-fed animal 18746 was fed with capsules containing glucose. Animal 18586 was fed with 1 mg glatiramer acetate in capsules with cracked glucose. Animal 18586 was fed with 1 mg glatiramer acetate in capsules with cracked enteric coating. Animal 18639 was fed with 20 mg glatiramer acetate in capsules with cracked enteric coating. Animal 18724 was fed with 1 mg glatiramer acetate in intact enteric coated capsules. Animal 18810 was fed with 10 mg glatiramer acetate in intact enteric coated capsules. Animal 18962 was fed with 20 mg glatiramer acetate in intact enteric coated capsules.

The schedule of feeding, disease induction and follow-up were substantially the same as those of Example 3 described hereinabove.

Results

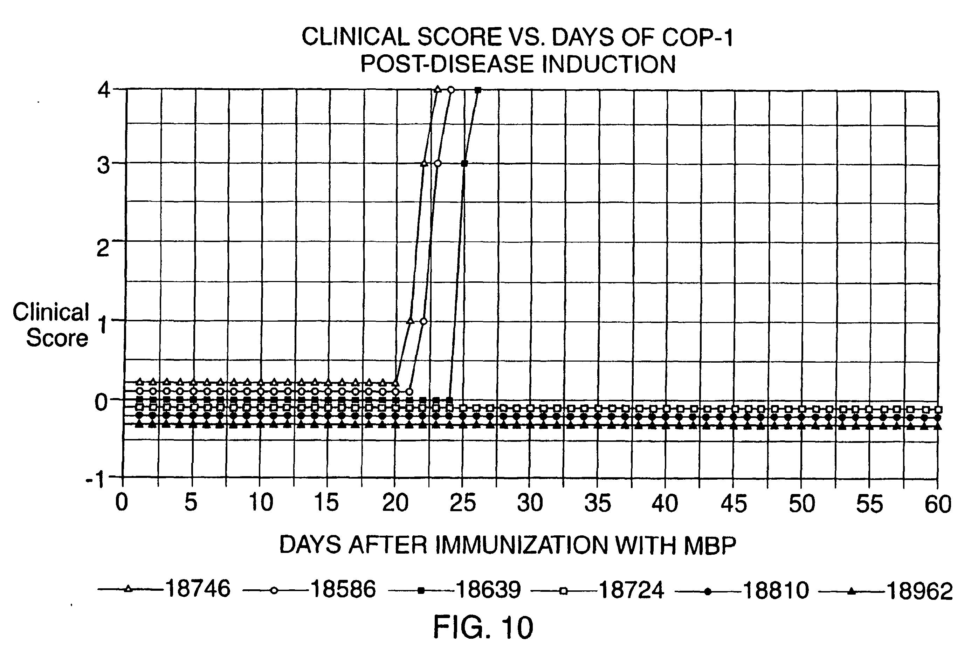

As can be seen with reference to Fig. 10, control monkey 18746 developed acute disease beginning on day +21 and died on day +23 of disease manifestations. Animals 18586 and 18639 treated with the "cracked" enteric coating, which opened in the stomach (at both doses), were not protected from the disease and died within 2-3 days of disease manifestations. All the monkeys fed with glatiramer acetate in enteric coated capsule were fully protected from the disease, and developed no signs of EAE until day 60, when they were sacrificed for histology.

**Claims**

1. Use of an amount from 0.1 mg to 1000 mg of glatiramer acetate in a solid form in the manufacture of an enterically-coated composition for oral administration to treat multiple sclerosis.

2. Use according to Claim 1, wherein the enterically-coated composition is an enterically-coated capsule.

3. Use according to Claim 1 or 2, wherein the amount is 1 mg, 10 mg or 20 mg.

4. A pharmaceutical composition comprising glatiramer acetate in solid form in an amount from 0.1 mg to 1000 mg in an enterically-coated composition.

5. The pharmaceutical composition of Claim 4, wherein the enterically-coated composition is an enterically-coated capsule.

6. The pharmaceutical composition of Claim 4 or 5, wherein the enteric coating comprises Eudragit S.

7. The pharmaceutical composition of Claim 4 or 5, wherein the enteric coating comprises Eudragit L.

8. The pharmaceutical composition of Claim 7, wherein the Eudragit L is L30D55.

**Patentansprüche**

1. Verwendung einer Menge von 0.1 mg bis 1000 mg an Glatiramer-Acetat in einer festen Form bei der Herstellung einer, mit magensaftresistenter Schutzhülle versehenen, Zusammensetzung zur oralen Verabreichung, um Multiple Sklerose zu behandeln.

**2.** Verwendung nach Anspruch 1, wobei die mit magensaftresistenter Schutzhülle versehene Zusammensetzung eine mit magensaftresistenter Schutzhülle versehene Kapsel ist.

**3.** Verwendung nach Anspruch 1 oder 2, wobei die Menge 1 mg, 10 mg oder 20 mg beträgt.

**4.** Pharmazeutische Zusammensetzung umfassend Glatiramer-Acetat in fester Form, in einer Menge von 0.1 mg bis 1000 mg, in einer mit magensaftresistenter Schutzhülle versehenen Zusammensetzung.

**5.** Pharmazeutische Zusammensetzung von Anspruch 4, wobei die mit magensaftresistenter Schutzhülle versehene Zusammensetzung eine mit magensaftresistenter Schutzhülle versehene Kapsel ist.

**6.** Pharmazeutische Zusammensetzung von Anspruch 4 oder 5, wobei die magensaftresistente Schutzhülle Eudragit S. umfasst.

**7.** Pharmazeutische Zusammensetzung von Anspruch 4 oder 5, wobei die magensaftresistente Schutzhülle Eudragit L. umfasst.

**8.** Pharmazeutische Zusammensetzung von Anspruch 7, wobei das Eudragit L. L30D55 ist.


**Revendications**

**1.** Utilisation d'une quantité de 0,1 mg à 1 000 mg d'acétate de glatiramère sous forme solide pour la fabrication d'une composition à enrobage entérique pour une administration orale pour traiter la sclérose en plaques.

**2.** Utilisation selon la revendication 1, dans laquelle la composition à enrobage entérique est une capsule entérosoluble.

**3.** Utilisation selon la revendication 1 ou 2, dans laquelle la quantité est 1 mg, 10 mg ou 20 mg.

**4.** Composition pharmaceutique comprenant de l'acétate de glatiramère sous forme solide en quantité de 0,1 mg à 1 000 mg dans une composition à enrobage entérique.

**5.** Composition pharmaceutique selon la revendication 4, dans laquelle la composition à enrobage entérique est une capsule à enrobage entérique.

**6.** Composition pharmaceutique selon la revendication 4 ou 5, dans laquelle le revêtement à enrobage entérique comprend de l'Eudragit S.

**7.** Composition pharmaceutique selon la revendication 4 ou 5, dans laquelle le revêtement à enrobage entérique comprend de l'Eudragit L.

**8.** Composition pharmaceutique selon la revendication 7, dans laquelle l'Eudragit L est L30D55.

EP 0 975 351 B1

FIG. 1

FIG. 2

FIG. 3

FIG. 4

Oral Supression of EAE in Mice by Cop1

FIG. 5

PROLIFERATION AND CYTOKINE SECRETION BY T-CELL LINE DERIVED
FROM SPLEENS OF COP1-FED RATS.

FIG. 6

INHIBITION OF EAE BY T-CELL LINE DERIVED FROM SPLEENS OF COP1 FED RATS

FIG. 7

INHIBITION OF EAE BY T-CELL LINE DERIVED FROM COP1 FED MICE

FIG. 8

FIG. 9

CLINICAL SCORE VS. DAYS OF COP-1
POST-DISEASE INDUCTION

Clinical Score

DAYS AFTER IMMUNIZATION WITH MBP

—△—18746 —○—18586 —■—18639 —□—18724 —●—18810 —▲—18962

FIG. 10